# EUROPÄISCHE PATENTANMELDUNG

(11) **EP 1 321 123 A1**
(43) Veröffentlichungstag der Anmeldung: **25.06.2003**
(21) Anmeldenummer: 01403313.8
(22) Anmeldetag: 20.12.2001
(51) Int. Cl.: A61K 7/06, A61K 7/42, A61K 7/48

(54) **Verwendung eines Extraktes der Pflanze Baptisia tinctoria in einer kosmetischen Zusammensetzung**

(71) Anmelder: Cognis France S.A., 31360 Saint-Martory (FR)
(72) Erfinder: Henry, Florence, 54600 Villers-Les-Nancy (FR); Contet-Audonneau, Jean-Luc, 54130 Saint-Max (FR); Danoux, Louis, 54420 Saulxures-Les-Nancy (FR); Pauly, Gilles, 54000 Nancy (FR)
(74) Vertreter: Herrburger, Pierre

(57) **Zusammenfassung**

Die Zielsetzung der vorliegenden Erfindung besteht in der Verwendung eines Wirkstoffes aus dem Extrakt der Wurzeln von Baptisia tinctoria in einer kosmetischen Zusammensetzung für die örtlich wirkende Anwendung auf der Haut, den Epithelanhanggebilden und/oder den Schleimhäuten.

## Beschreibung

### Gebiet der Erfindung

Die vorliegende Erfindung bezieht sich auf das Gebiet der Kosmetik. Ihre Zielsetzung besteht in der Verwendung von mindestens einem Extrakt der Baptisia tinctoria-Pflanze. Ein weiterer Gegenstand der Erfindung sind kosmetische Zubereitungen, die einen derartigen Extrakt enthalten.

### Stand der Technik

Baptisia tinctoria (syn. Sophora tinctoria) , auch wilder Indigo genannt, aus der Familie der Fabaceae ist ein mehrjähriges bis zu 60 cm hoch wachsendes Kraut mit kleinen dreigeteilten Blättern und kleinen gelben Blüten, das in Wäldern oder vorwiegend an Waldrändern wächst. Der Ursprung dieser Pflanze liegt im mittleren Westen der USA, North Carolina, West Virginia bis Florida und India bis Minnesota.

Schon im letzten Jahrhundert wurden die in Wasser gekochten Wurzeln von Baptisia tinctoria innerlich als Heilmittel gegen Fieber, Scharlach, Typhus und Halsbeschwerden eingesetzt. Äußerlich wurden Geschwüre und offene Wunden adstringierend und antiseptisch mit dem wilden Indigo behandelt.
Auch heute ist der pharmazeutische Gebrauch von Extrakten der Wurzeln in Kombination mit weiteren Heilpflanzen wie Echinacea und Thuja durch den Einsatz in Produkten zur Immunsystemstärkung in der traditionellen Medizin weit verbreitet, Man schreibt den Polysacchariden und Proteinen von Baptisia die immunstärkende Wirkung zu.
In der internationalen Patentanmeldung WO 99/34811 A1 werden Mischungen aus Pflanzenextrakten, die unter anderem Baptisiaextrakte enthalten auch als antifungistische Zubereitungen offenbart.

Zu den bioaktiven Wirkstoffen, die in der Pflanze nachgewiesen wurden, zählen Biochanin A, Cytisin, Daidzin, Formonetin, Fustin, Garbanzol, Ononin, Orobol, Pseudobaptigenin, Scopolin, Sparteine, Tectoridin und Tectorigenin. [Phytochemical dictionary. A handbook of bioactive compounds from plants, Harborne, J.B, et al., 1999, S. 976]. Die Wurzeln enthalten ferner Arabinogalactane (Heteroglycane mit einem Molekulargewicht von 25000-500000), Glycoproteine, Flavone und Isoflavone aus der Gruppe der Apigenine, Apigénine 7-0 rhamnoglucoside, Lutéoline, Lutéoline 7-0 glucoside, Lutéoline 7-0 rhamnoglucoside. Génistéine, Génistéine 7-0-rhamnoglucoside, Biochanine A,biochanine A 7-0 rhamnoglucoside, Orobol,Orobol 7-0 rhamnoglucoside, Tectorigénine, Tectorigénine 7-0 glucoside, Dadzeine, , Formonétine, Formonétine 7-0 glucoside, Formonétine 7-0 rhamnoglucoside, Pseudobaptigénine, Pseudobaptigénine 7-0 rhamnoglucoside. Coumarine ; Scopolétine und Scopolétine 7-0 glucoside. **Distribution of flavonoids in the genus baptisia (Leguminosae), Mabry, T.J. et Swift, W. T., Phytochemistry, 1970, 9, S, 2359-2364.**

In Anlehnung an pharmazeutische Indikationen wird auch auf dem kosmetischen Markt nach pflanzlichen Wirkstoffen gesucht, die beispielsweise für die Haut pflegende, vor Alterserscheinungen schützende, und revitalisierende Eigenschaften vermitteln. Die Zusammensetzung des Produktes soll dabei zusätzlich eine optimale dermatologische Verträglichkeit besitzen, so dass auch empfindliche Verbraucher nicht mit Irritationen reagieren. Darüber hinaus sollten die Mittel jedoch auch weitere Funktionen erfüllen, die gleichzeitig die technischen Eigenschaften des kosmetischen Produktes, wie Lagerstabilität, Lichtstabilität und Formulierbarkeit positiv beeinflussen oder zumindest nicht verschlechtern.

Die komplexe Aufgabe der vorliegenden Patentanmeldung hat darin bestanden, neue Wirkungen der bereits bekannten Pflanze zu finden und die Verwendung dieser Extrakte in kosmetischen Mitteln zu ermöglichen, die sich durch eine hohe Verträglichkeit auch für empfindliche Haut auszeichnen und zusätzlich eine gute physiko-chemische Stabilität aufweisen.

### Beschreibung der Erfindung

Gegenstand der vorliegenden Erfindung ist die Verwendung eines aus der Pflanze Baptisia tinctoria extrahierten Extraktes zur Herstellung kosmetischer Zubereitungen für die lokale Anwendung auf der Haut, den Epithelanhanggebilden und/oder den Schleimhäuten.
Ein weiterer Gegenstand der Erfindung sind kosmetische Zubereitungen für die lokale Applikation, enthaltend einen Extrakt der Wurzeln von Baptisia tinctoria.

Es wurde überraschender Weise festgestellt, dass die Extrakte von Baptisia tinctoria bei lokaler Anwendung auf der Haut, den Epithelanhanggebilden und den Schleimhäuten besondere biologische Wirkungen hervorrufen und über eine sehr gute Toleranz verfügen und dass die Verwendung als Wirkstoff, alleine oder in Verbindung mit mindestens einem anderen Wirkstoff, von mindestens einer aus Baptisia tinctoria extrahierten Fraktion in einer Zusammensetzung oder einem kosmetischen Erzeugnis bei lokaler Anwendung auf der Haut, den Epithelanhanggebilden und/oder den Schleimhäuten es ermöglicht, regenerative Wirkungen, Anti-Reizwirkungen, Anti-Aging Effekte, UV-Schutzwirkung, schützende Wirkung gegen oxidativen Strress und anti-inflammatorische Aktivitäten, sowie lipolytische Aktivitätan humanen Fettzellen festzustellen. Die Extrakte von Baptisia tinctoria zeichnen sich besonders zur vorbeugenden und heilenden Behandlung empfindlicher Hauttypen und als Sonnenschutzmittel aus. Sie werden hervorragend vertragen.
Als Wirkstoff im Sinne der vorliegenden Erfindung werden Extrakte der Pflanze Baptisia tinctoria, insbesondere Extrakte der Wurzeln von Baptisia tinctoria verstanden. Dabei sind ausgeprägte Wirkungen besonders den (Iso)flavonhaltigen Auszügen der Wurzeln zuzuschreiben. Bei den extrahierten Fraktionen, die den Wirkstoff bilden, werden Zusammensetzungen die mindestens zwei Isoflavonfraktionen (Tabelle 1) enthalten speziell bevorzugt.

Die Extrakte nach der Erfindung können auch in jeden anderen zutreffenden kosmetischen Vektor eingearbeitet werden oder mit ihm verbunden werden, zum Beispiel filmbildende Mittel, Liposome, Zyklodextrine, Micellen, Makro-, Mikro- und Nanopartikel, sowie Makro-, Mikro und Nanokapseln oder sie können absorbiert oder auf organische Polymere oder mineralische Träger verpflanzt werden.
Zu den bekannten Inhaltsstoffen des Extraktes von Baptisia tinctoria zählen Isoflavone, Flavone, Proteine, Glycoproteine und Kohlenhydrate, insbesondere sind das unter den Flavonen : Apigenine, Apigenine 7-0 rhamnoglucoside, Luteoline, Luteoline 7-0 glucoside, Luteoline 7-0 rhamnoglucoside, unter den Isoflavonen: Genisteine, Genisteine 7-O-rhamnoglucoside, Biochanine A, Biochanine A 7-O Rhamnoglucoside, Orobol, Orobol 7-0 rhamnoglucoside, Tectorigenine, Tectorigenine 7-0 glucoside, Dadzeine, Formonetine, Formonetine 7-0 glucoside, Formonetine 7-0 rhamnoglucoside, Pseudobaptigénine, Pseudobaptigénine 7-0 rhamnoglucoside und die Coumarine : Scopoletine und Scopoletine 7-0 glucoside.

### Gewinnung des Extraktes von Baptisia tinctoria

Die Herstellung der erfindungsgemäß einzusetzenden Extrakte erfolgt durch übliche Methoden der Extraktion von Pflanzen bzw. Pflanzenteilen, Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der **Pharmazeutischen Praxis,** (5. Auflage, Bd. 2, S, 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von Pflanzen und/oder Pflanzenteilen ausgegangen, die vor der Extraktion mechanisch zerkleinert und gegebenenfalls entfettet werden. Hierbei eignen sich alle dem Fachmann bekannten Zerkleinerungsmethoden, als Beispiel sei die Zerkleinerung mit einem, mit Messern versetzten enthaltenen Gerät genannt. Zur Extraktion besonders bevorzugt sind die Blätter der Pflanze.
Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Bevorzugt ist die Extraktion mit Wasser, Methanol, Ethanol, Propanol, Butanol und deren Isomere, Aceton, Propylenglycolen, Polyethylenglycolen Ethylacetat, Dichlormethan, Trichlormethan sowie Mischungen hieraus. Die Extraktion erfolgt in der Regel bei 20 bis 100 °C, bevorzugt bei 80 bis 100°C, insbesondere bei 80 bis 90°C. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 5 bis 30, vorzugsweise 10 bis 20, insbesondere 12 bis 18 Gew,-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.
Der Wirkstoffgehalt im Sinne der Erfindung bezeichnet die Summe aller im Extrakt vorhandenen Wirkstoffe bezogen auf das Trockengewicht des Extraktes.
Wirkstoff im Sinne der Erfindung bezieht sich auf die im Extrakt enthaltenen Inhaltstoffe auch wenn deren Gehalt und Identität mit herkömmlichen, dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.
Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

### Bevorzugte Methode - Extraktion:

Gepulverte Wurzeln von Baptisia tinctoria werden mit der 5 bis 20 fachen, vorzugsweise 8 bis 12fachen Menge ihres Gewichtes an Extraktionsmedium versetzt und unter Rühren für 1 bis 5, vorzugsweise 2 bis 3 Stunden extrahiert. Als Extraktionsmedium wird besonders bevorzugt Methanol in Konzentrationen von 40 bis 98 Vol. %, vorzugsweise 50 bis 90 Vol.%, besonders bevorzugt 60 bis 80 Vol.% eingesetzt. Der Extrakt kann als wäßrig und/oder alkoholische Zubereitung eingesetzt werden oder nachfolgend getrocknet werden nach den herkömmlichen Trocknungsverfahren.

### Methode - Hydroponie:

Eine weitere Methode zur Gewinnung primärer und sekundärer Metaboliten aus Baptisia tinctoria ist die Kultivierung unter Hydroponie - Bedingungen. Diese wird entsprechend der in der internationalen Anmeldung WO 01/33942 A2 beschriebenen Methode durchgeführt.

### Beispiel 1 - Alkoholische Extraktion

0,075 kg der gepulverten Wurzeln von Baptisia tinctoria wurden in einem Rührgefäß mit 750 ml 80 Vol.% Methanol versetzt. Die Mischung wurde unter Rückfluß zwei Stunden gerührt. Danach wurde der feste Rückstand durch Filtration entfernt und das Methanol aus der gefilterten Lösung durch Evaporieren entfernt. Der wäßrige Extrakt wurde 15 min. (4200 trs/min) zentrifugiert und nachfolgend gefriergetrocknet. Ausbeute der Extraktion: 15.92 Gew. % - bezogen auf die Einsatzmenge der gepulverten Wurzeln.

### Fraktionierung des Extraktes aus Beispiel 1 zur Aufreinigung der Isoflavone

20 g des Extraktes nach Beispiel 1 wird chromatographisch durch unterschiedliche Konzentrationsgradienten an Methanol eluiert. Das bevorzugte Adsorbens hat Polymerstruktur wie Ambertite oder reversed phase C18 - Silicagel.
Die Moleküle mit niedriger Affinität zum Adsorbenten werden gering adsorbiert bzw. vornehmlich desorbiert, passieren direkt die Matrix und werden im Waschwasser hinter der ersten Waschschritt-Säule gesammelt. Ein stufenweiser Gradient aus Wasser-Methanolschritten wird mit einer Flußrate von 10 ml/min, zur Desorption eingesetzt: 4 Liter Wasser, darauffolgend 4 Liter Methanol 20% (v/v), 4 Liter Methanol 40% (v/v), 4 Liter Methanol 60% (v/v), 4 Liter Methanol 80% (v/v), und 4 Liter reines Methanol.

**Tabelle 1:**

| **Desorption der Isoflavone nach der angegebenen Rate :** | | |
|---|---|---|
| **Eluent** | **Eluierte Substanz** | **Ausbeute der getrockneten Fraktion (weight fraction/ weight raw extract)** |
| Wasser | Polare Alkaloide | 41.49 % |
| Methanol 20% | Polare Alkaloide | 1.12 % |
| Methanol 40% | Polare Alkaloide | 3.58 % |
| Methanol 40% und 60%* | Polar alcaloides, Daidzein glycoside, Genistein glycoside | 3.51 % |
| Methanol 60% | Polare Alkaloide, Daidzein glycoside, Genisteine glycoside, Formonétine glycoside | 4,03 % |
| Methanol 60% und 80%* | unpolare Alkaloide Daidzeine glycoside, Genistein glycoside, Formonetine, Biochanine glycoside | 22.76 % |
| Methanol 80% | unpolare Alkaloide Dadzein, Genistein glycoside ;Formonetin glycoside, Biocahnine glycoside | 3.20% |
| Methanol 80% | Unpolare Alkaloide, Dadzein, Genistein, Formonetin glycoside, Biochanine glycoside | 1.48% |
| Methanol 80% und Methanol 100%* | unpolare Alkaloide Genistein, Formonetin , | 3.26 % |

| | | |
|---|---|---|
| * Zwei Fraktionen entsprechend ihres chromatographischen Profils zusammengefügt Das Lösungsmittel jeder Fraktion wurde abgedampft und die Fraktionen gefriertrocknet und analysiert resp. ausgewogen. | | |

### Beispiele für den Nachweis der Eigenschaften der Extrakte von Baptisia tinctoria

Die biologischen Eigenschaften und Aktivitäten des Extraktes aus den Wurzeln der Baptisia tinctoria konnten durch Tests bestimmt und gemessen werden, die dem Fachmann auf dem Gebiet bekannt sind, ihre Ergebnisse werden nachstehend aufgeführt:

### 1. Zellüberlebens - Test

Für die Beurteilung der Zellaktivität gibt es wesentliche Marker, zu denen ATP, Proteine und Glutathion zählen.
ATP (Adenosintriphosphat) ist ein Zellbestandteil, der Energie speichert und vorwiegend in Mitochondrien produziert wird. Zellen benötigen ATP, um die Aktivität ihrer Enzyme zu sichern, die wiederum das Cytoskellet, die lonenkanäle, die Nahrungsaufnahme und viele lebenswichtige biologische Prozesse der Zelle steuern.
(Vasseur P., Aerts C. Appréciation de la cytotoxicité par la mesure de l'ATP. Journal français Hydrologie (1982) vol 9, pp 149-156).
Die Proteinkonzentration der Zellen wurde bestimmt nach Bradford (Bradford M.M. A rapid and sensitive method for the quantitation of microgram quantities of protein utilizing the principle of protein-dye binding. Anal. Biochem. (1977) vol 72, pp 248-254)
Glutathion (GSH) ist ein Peptid, das von Zellen produziert wird, um die Zelle vor oxidativem Stress oder Schwermetallen wie beispielsweise Blei oder Quecksilber zu schützen. Die drei Aminosäuren die bei der reduzierten Form von GSH involviert sind, sind wiederum verbunden mit spezifischen cytoplasmatischen Enzymen, die ATP benötigen.
Die Steigerung des GSH-Levels hat einen positiven Einfluß auf die Aktivität der Glutathion-Stransferase, die ein entgiftendes Enzym darstellt.
GSH wurde bestimmt nach Hissin (Hissin P.J., Hilf R. A fluorometric method for determination of oxidised and reduced Glutathione in tissues. Analytical Biochemistry (1977) vol 74, pp 214-226).

### 1) WIRKUNGEN AUF DAS ÜBERLEBEN HUMANER FIBROBLASTEN (Tabelle 1)

Menschliche Fibroblaste werden in ein Nährmedium (DMEM = Dulbecco Minimum Essential Medium von der Firma Life Technologie Sarl) mit und ohne 10 % fötalem Kälberserum (von der Firma Dutcher) geimpft und 24 Stunden lang bei 37° C in einer 5 %igen CO₂-Athmosphäre inkubiert. Dieser Test wurde im Vergleich mit und ohne fötales Kälberserum durchgeführt.

Das Wachstumsmedium wird danach durch ein Sub-Optimum-Medium (ohne FCS) ersetzt, das verschiedene Konzentrationen des Extraktes (0 = Kobtrolle; 0,01, 0,03 und 0,1 % Gew./Vol., Gewicht/Volumen) nach der Beschreibung Beispiel 1 enthält. Nach einer 3tägigen Inkubation bei 37°C wurde das Wachstum durch eine Zählung der haftenden Zellen durch einen automatischen Partikelzähler und durch die Dosierung der intrazelluläre ATP-Gehalt ausgewertet.

Das Überleben wurde durch die Dosierung der folgenden Gehalte ausgewertet:
- Rate des metabolisierten MTT (Methyl Thiazolyl Tetrazolium)
Die Aktivität der Mitochondrien wird über den MTT-Test bestimmt. MTT wird durch ein Enzym der Respirationskette, Succinatdehydrogenase, in Formazan reduziert (Denizot F, Lang R, Rapid colorimetric assay for cell growth and survival. J. Immunol. Methods, 89, 271-277, 1986).
- von Proteinen,
- von Glutathion (GSH), ein direkt von der Zelle erzeugtes Peptid, zur Bekämpfung von oxydativem Stress oder von verschiedenen Schmutzstoffen, wie zum Beispiel Schwermetalle. Seine Synthese erfordert ATP als Energiequelle.

Die Versuche wurden in dreifacher Ausführung vorgenommen und zwei- oder dreimal wiederholt. Die in unterschiedlichen Konzentrationen eingesetzten Extrakte von Baptisia tinctoria wurden hergestellt nach dem Verfahren Beispiel 1,
Die Ergebnisse werden im Verhältnis zu einer extraktfreien Formulierung für Protein, MTT und GSH in das Verhältnis gestellt und in einem Prozentsatz im Verhältnis zum unbehandelten Kontrollmittel angegeben als Durchschnittswert +/- SEM (Fehlertyp des Durchschnitts) ausgedrückt.

Die Tabelle 1 gibt jeweils die Mitochondrienaktivität über die MTT, Proteingehalte und die GSH-Gehalte an, die nach drei Tagen für verschiedene Konzentrationen an Extrakten ( 0, 0,01 und 0,03 % Gew./Vol.) gemessen wurden. Ein Extrakt der Baptisia tinctoria hat mit einer Konzentration ab 0,01 Gew.% die Mitochondrienaktivität danach erheblich erhöht. Die Ergebnisse zeigen, dass auch der Gehalt an synthetisierten Proteinen und Glutathion deutlich gesteigert wurde.

Diese Ergebnisse beweisen, dass die (Iso)flavonhaltigen Extrakte der Wurzel von Baptisia tinctoria hohe Fähigkeiten zur Verbesserung des Wachstums und des Metabolismus (Synthese von ATP, der Proteine und des Glutathion) der menschlichen Fibroblaste aufweisen, was deutlich eine energiespendende, stimulierende und "Anti-Älterungs"- Aktivität dieser Extrakte angibt.

**Table 1:**

| **Zellüberlebens - Test** | | | |
|---|---|---|---|
| Ergebnisse in % basierend auf der Kontrolle ohne Extrakt (Mittelwert von 3 Assays in dreifacher Ausführung) | | | |
| Ergebnisse ohne fötales Kälberserum | | | |
| Konzentration des Extraktes % Gew./Vol. | MTT | Proteine | GSH/Proteine |
| 0 = Kontrolle | 100 | 100 | 100 |
| 0,01 | 106 | 102 | 133 |
| 0,03 | 108 | 106 | 210 |
| | | | |

| Ergebnisse mit fötalem Kälberserum | | | |
|---|---|---|---|
| Konzentration des Extraktes % Gew./Vol. | MTT | Proteine | GSH / Proteine |
| 0=Kontrolle | 100 | 100 | 100 |
| 0,01 | 119 | 105 | 135 |
| 0,03 | 132 | 110 | 188 |

### 2. Test zur Stimulation von Glucosaminoglucanen (GAGS)

Als Bestandteile der Haut sind verschiedene Grundstrukturen zu unterscheiden wie Zellen (Fibroblasten, Mastzellen), Gewebefasern (Kollagen und Elastin) und dermale Makromoleküle. Als dermale Makromoleküle sind im Sinne der Erfindung prinzipiell alle Makromoleküle zu verstehen, die als Bestandteile der Haut entweder in der Basalmembran zwischen Dermis und Epidermis oder in der Dermis und Epidermis direkt zu finden sind. Es handelt sich bei den dermalen Makromolekülen im Besonderen um solche, die ausgewählt sind aus der Gruppe, die gebildet wird aus Glykosaminoglykanen insbesondere Chondroitinsulfat, Keratansulfat, Dermatansulfat und Hyaluronsäure und deren Salze, Collagen insbesondere Collagen Typlll, Elastin, Fibronectin, Proteoglycanen und deren Salze.
Die **Glykosaminoglykane** werden auch als Mucopolysaccharide bezeichnet. Es handelt sich um negativ geladene, lange unverzweigte Polysacharide (Glykane), welche aus 1,4-verknüpften Einheiten von Dissachariden bestehen, in denen ein Mol einer Uronsäure (D-Glucuronsäure oder beispielsweise L-Iduronsäure) mit der 3-Stellung eines N-acetylierten Aminozuckers (Glykosamins) glykosisdisch verbunden sind. Die Glykosaminoglykane sind im Gewebe zu mehreren Ketten an ein Kern-Protein (core protein) gebunden und bilden so die Proteoglykane. Das **Chondrotinsulfat** zählt zu den Glykosaminoglykanen. Es kommt im Gewebe als Chondroitin-4-sulfat oder als Chondroitin-6-sulfat vor und besteht u.a aus D-Glucuronsäure und N-Acetyl-D-galactosamin. Die molare Masse beträgt zwischen 5000-50000. Das auch als beta-Heparin bezeichnete, nicht gerinnungshemmend wirksame Glycosaminoglykan **Dermatansulfat** besteht aus L-Iduronsäure oder D- Glucuronsäure, N-Acetyl-D-galactosamin und Sulfat-Gruppen. Die molare Masse von Deramtansulfat liegt zwischen 15000 und 40000. Die **Hyaluronsäure** ist ein saures Glykosaminoglykan, Grundbaustein der Hyaluronsäure ist ein aus D-Glucuronsäure und N-Acetyl-D-glucosamin in (beta 1-3)-glykosidischer Bindung aufgebautes Aminodisaccharid, das mit der nächsten Einheit (beta 1-4)-glykosidisch verbunden ist. Die Hyaluronsäure trägt im Gegensatz zu vielen anderen Glykosaminoglykanen keine Sulfatgruppen und ist nicht proteingebunden im Gewebe.
**Collagen** besteht aus Proteinfasern und kommt in menschlicher Haut in drei verschiedenen Typen (Typ I, III und IV) vor. Im Collagen sind die einzelnen Polypeptidketten, die jeweils viel von der Aminosäure Prolin und als jeden dritten Rest Glycin enthalten, umeinander zu einer Tripelhelix gewunden. Die Collagenfasern werden als Tropokollagen in den Fibroblasten synthetisiert und in die extrazelluläre Matrix ausgeschleust. Die erfindungsgemäße Stimulierung der Synthese des Collagens führt zu einer Erhöhung der Produktion an Collagen und damit zu einer erhöhten intermolekularen Verfestigung der Dermis und dadurch zu einer straffer erscheinenden Haut, Das **Elastin** ist ebenfalls ein faserartiges Protein. Hierbei handelt es sich um unstrukturierte kovalent quervernetzte Polypeptidketten, die ein gummiähnliches elastisches Material bilden. Das Elastin wird nach der Synthese in den Hautzellen in die extrazelluläre Matrix ausgeschleust. Die erfindungsgemäße Stimulierung der Synthese der Elastin-Polypeptidketten führt zu einer Erhöhung der Produktion an Elastin und damit zu einer Erhöhung der Elastizität der Haut.
**Fibronectin** stellt eine Gruppe hochmolekularer Glykoproteine (MR des Dimers ca. 440 000-550 000) dar, die sich in der extrazellulären Matrix und in extrazellulären Flüssigkeiten finden. Das durch zwei Disulfid-Brücken verbundene Fibronectin-Dimer, ein langgestrecktes Molekül mit den Abmessungen 600x25 Å, bindet durch lineare Kombination dreier verschiedener sich wiederholender Domänen u. a. Collagene, Glykosaminoglykane, Proteoglykane, Fibrin(ogen), Desoxyribonucleinsäuren, Immunglobuline, Plasminogen, Plasminogen-Aktivator, Thrombospondin, Zellen und Mikroorganismen. Durch diese Eigenschaften vermittelt es z. B. die Anhaftung von Bindegewebszellen an Collagen-Fibrillen oder von Thrombocyten und Fibroblasten an Fibrin (Beitrag zur Wundheilung).
Die **Proteoglycane** bestehen wie die Glycoproteine aus Kohlenhydraten und aus Proteinen, bei den Proteoglycanen überwiegt jedoch der Anteil an Polysacchariden. Die Proteoglycane der Haut enthalten Dermatansulfat. Es lagern sich ca. 140 solcher Proteoglycane mit Hilfe kleinerer Proteine (Link-Proteine) nichtkovalent an eine Hyaluronsäure-Kette zu Molekül-Aggregaten mit einer mittleren Molmasse von ca. 2 Mio. an. Die durch ihr Wasserbindevermögen ausgezeichneten polyanionischen Aggregate können feste Gele bilden, die dem Stützgewebe (extrazelluläre Matrix) Elastizität und Zugfestigkeit verleihen. In Schleimen schützen sie die Epithelien. Die erfindungsgemäße Stimulierung der Synthese von Proteoglycanen und Hyaluronsäure führt zu einer größeren Menge an extrazellulärer Matrix und damit zu einer erhöhten Elastizität und Zugfestigkeit.

Während der Alterung nimmt die Dicke der Haut, die Durchblutung und auch die Anzahl der Fibroblasten ab. Kollagenstränge werden durchlässiger und ungeordneter und die Rate des unlöslichen Kollagens steigt auf Kosten des löslichen. Ebenso werden feine elastische Fasern grob oder lösen sich gänzlich auf. Außerdem ist die Synthese von Glucosaminoglucanen deutlich vermindert.

Zur Bewertung von Substanzen mit Anti-Aging-Wirkung wurde daher ein Modell entwickelt, das es ermöglicht, die Stimulation der Synthese dermaler Makromoleküle nachzuweisen.
Dieses Modell basiert auf einer Kultur von Fibroblasten mit Kollagen Typ 1, die Kollagengewebe aufbauen. In den Schnitten der Kollagengewebe werden die zu untersuchenden Makromoleküle durch Anfärbetechniken oder immunohistochemische Verfahren bestimmt und durch Bildanalyse quantifiziert.
Diese Methode ermöglichte es, den stimulierenden Effekt von Baptisia-Extrakten auf die Synthese dermaler Makromoleküle (Elastin, Kollagen Typ III und GAGS als Chondroitinsulfat) durch Fibroblasten darzustellen.

### Methode:

Eine Zellsuspension humaner Fibroblasten wurde gemischt mit einer Lösung von Kollagen Typ I(1 - 2 mg/ml). Diese Mischung wurde in einem definiertem Nährmedium (DMEM = Dulbecco Minimum Essential Medium, Firma Life Technologie Sarl) mit 2 Gew.-% fötalem Kälberserum (FCS) bei 37°C in einer 5%igen CO₂-Atmosphäre in Petri-Schalen (5ml/Schale) unter Zusatz verschiedener Konzentrationen der zu untersuchenden Pflanzenextrakte über 7 Tage inkubiert.
Dem fötalen Kälberserum (FCS) waren Lipide entzogen durch Aktivkohle SIGMA oder Folch Methode (A simple method for the isolation and purification of total lipids from animal tissues, Folch at al., J. Biol. Chem., 1954, Vol. 226, S. 497-509).
Nach der Inkubationszeit von 7 Tagen wurden Biopsien (Gewebeproben) genommen und histologische Schnitte des Collagen-Gels mit humanen Fibroblasten erhalten.. Die Synthese der Makromoleküle wurde durchgeführt durch Anfärbung der GAGS mit PAS-Alcian Blau (Standardfärbemethode. z.B. von der Firma SIGMA nach der Periodic-Acid-Schiff-Methode (PAS), beschrieben in: Mowry RW, Anal. NY Adad. Sci. 106 Art 2, 402, 1963).
Bilder wurden für jede Bedingung mit der Kamera aufgenommen. Die Auswertung der Synthese der Makromoleküle erfolgte dann im Bereich um die Fibroblasten, auch "perifibroblastic area" genannt.
Zur Quantifizierung mit Bildanalyse wurden die reaktiven Strukturen in diesem Bereich und die unterschiedlichen Grau-Stufen aufgenommen. Beide Parameter sind direkt proportional zu der Intensität der Makromolekülsynthese der Fibroblasten.
Sie werden in den Ergebnissen (Tabelle 2) als "Synthesefaktor der Fibroblasten" in einem Wert dargestellt und repräsentieren die Syntheseaktivität der Fibroblasten (Mittelwert aus zwei Versuchsdurchführungen ± Standardabweichung SEM).

### Ergebnisse:

**Tabelle 2:**

| **% an Verbesserung des Fibroblastensynthesefaktors bezogen auf die Kontrolle ohne Baptisia Extrakt (t=** 7 **Tage)** | |
|---|---|
| **Bedingungen** | **Ergebnisse in % gegen Kontrolle** |
| FCS entfettet durch Aktivkohle + 0,01 Gew.% Extrakt nach Beispiel 1 | 35.17 |
| FCS entfettet durch Aktivkohle + 0,03 Gew.% Extrakt nach Beispiel 1 | 43.29 |
| FCS entfettet durch Folch Methode + 0,01 Gew.% Extrakt nach Beispiel 1 | 15.45 |
| FCS entfettet durch Folch Methode + 0,03 Gew.% Extrakt nach Beispiel 1 | 19.16 |

Die dargestellten Ergebnisse machen einen signifikanten stimulierenden Effekt des Extraktes von Baptisia tinctoria auf die Synthese dermaler Makromoleküle durch Fibroblasten deutlich.
Es läßt sich daraus wiederum ein eindeutiger Anti-Aging-Effekt des untersuchten Extraktes ableiten.

### 3. Untersuchungen zum Nachweis des Zellschutzes vor toxischen Einwirkungen durch UV-Strahlung

### 3a) Test an humanen Fibroblasten nach UVA-Bestrahlung in vitro

UVA-Strahlen dringen bis in die Dermis ein, wo sie zu Oxidationsstreß führen, was durch eine Lipoperoxidation der Zytoplasmamembranen nachgewiesen wird.
Die Lipoperoxide werden zu Malonaldialdehyd abgebaut, der viele biologische Moleküle wie Proteine und Nukleinbasen vernetzen wird (Enzymhemmung bzw. Mutagenese).
Glutathione (GSH) ist ein Peptid, welches direkt von den Zellen produziert wird um oxidativem Stress oder schädigenden Umwelteinflüssen wie zum Beispiel einer erhöhten Quecksilber- oder Bleibelastung entgegen zu wirken. Der verbleibende Gehalt an GSH nach der Bestrahlung mit UVA-Strahlung wurde bestimmt nach der Methode von Hissin, beschrieben in Anal. Biochem., 74, 214-226, 1976.

### Methode:

Zur Durchführung dieser Tests wurde ein definiertes Kulturmedium (DMEM) mit 10 % fötalem Kälberserum mit den Fibroblasten beimpft und der Pflanzenextrakt (in dem definierten Medium mit 2 % Serum) 72 Stunden nach dem Beimpfen zugegeben,
Nach 48stündiger Inkubation bei 37 C und einem CO₂-Gehalt von 5 % wurde das Kulturmedium durch eine Saline-lösung ersetzt und die Fibroblasten wurden mit einer UVA-Dosis bestrahlt (20 J/cm²; Röhren: MAZDA FLUOR TFWN40).
Nach der Beendigung der Bestrahlung wurde der Gehalt an Zellproteinen und der Anteil an GSH bestimmt sowie der MDA-Spiegel (Malonaldialdehyd-Spiegel) in der überstehenden Saline-lösung quantitativ durch Reaktion mit Thiobarbitursäure bestimmt, Die Ergebnisse sind angegeben in Prozent im Vergleich zur Kontrolle ohne Bestrahlung.

**Tabelle 3a:**

| **Quantifizierung von Malonaldialdehyd, Zellproteinen und GSH in Fibroblasten (Ergebnisse in % bezogen auf die Kontrolle, Mittelwert aus 2 Versuchen** | | | |
|---|---|---|---|
| | freigesetztes MDA | Zellproteine | GSH / Protein |
| Kontrolle ohne UV-Bestrahlung without UV | **0** | **100** | **100** |
| UVA 20 J/cm2 | **100** | **99** | **75** |
| UVA + 0.0003 Gew. % Extrakt nach Beispiel 1 | **26** | **98** | **76** |
| UVA + 0,01 % Gew.% Extrakt nach Beispiel 1 | **73** | **107** | **70** |
| UVA + 0.03 %Gew.% Extrakt nach Beispiel 1 | **56** | **116** | **105** |
| UVA + 0,06 % Gew.% Extrakt nach Beispiel 1 | **47** | **111** | **113** |
| UVA + 0,1 % Gew.% Extrakt nach Beispiel 1 | **37** | **113** | **97** |

Die Ergebnisse zeigen eine dosisabhängige Abnahme der MDA-rate durch den Einsatz von Baptisia tinctoria - Extrakt was auf eine deutliche Verringerung der Lipoperoxidation durch UV-induzierten Oxidationsstreß hinweist.
Die Produktion von GSH , das dem oxidativeb Stress entgegenwirkt, wird bei Einsatz des Extraktes ab einer Konzentration von 0,03 Gew.% um ca. 25 % erhöht.
Diese Ergebnisse zeigen eine hohe Kapazität von Extrakten aus den Wurzeln von Baptisia tinctoria schädigende Effekte von oxidativem Stress auf der Haut zu reduzieren.

### 3b) Entzündungshemmende Eigenschaften in vitro - UVB Lichtschutz

UVB-Strahlen lösen durch Aktivierung eines Enzyms, nämlich Phospholipase A2 (PLA2) die Bildung von Arachidonsäure und weiteren ungesättigten C20-Säuren aus. Diese greifen durch die Cyclooxigenase über cyclische Endoperoxide in die Prostaglandinsynthese ein, die wiederum an der Entstehung von Entzündungen, Fieber und Schmerzen wesentlich beteiligt sind und eine Zellmembranschädigung verursachen.

### Methode:

Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Pflanzenextrakt von Baptisia tinctoria sowie zur vergleichenden Darstellung zwei handelsübliche antiinflammatorische Wirkstoffe (Acetylsalicylsäure bzw. Indomethacin) 72 Stunden nach dem Beimpfen zugegeben.
Die Keratinozyten wurden sodann mit einer UVB-Dosis bestrahlt (30 mJ/cm² - Röhren: DUKE GL40E).

Nach weiterer eintägiger Inkubation bei 37°C und bei 5 % CO₂ wurde der LDH- und der PGE2-Gehalt im Überstand bestimmt. Der Gehalt von LDH-(Lactatdehydrogenase) wurde mittels einer Enzymreaktion bestimmt (verwendeter Kit zur Untersuchung des LDH Gehaltes von der Firma Roche) Der Gehalt an PGE2 wurde mit einem ELISA-Test (ELISA Kit der Firma Roche) bestimmt.

**Tabelle 3b:**

| **Zellschutzwirkung eines Extraktes von Baptisia tinctoria gegen UVB-Strahlen; Ergebnisse in % bezogen auf die Kontrolle** | | | |
|---|---|---|---|
| Bedingung | Anzahl Keratinocyten | Gehalt an freigesetztem LDH | Gehalt an freigesetztem PGE2 |
| Kontrolle ohne UV | 100 | 0 | 0 |
| Kontrolle mit UVB (30 mJ/cm²) | 36 | 100 | 100 |
| UVB + 0,01 % Extrakt gemäß Beispiel 1 | 61 | 34 | 18 |
| UVB + 0,02 % Extrakt gemäß Beispiel 1 | 75 | 18 | 14 |
| UVB + 0,03 % Acetylsalicylsäure | 65 | 22 | 11 |
| UVB + 0,001 % Indomethacin | 36 | 74 | 18 |

Die Ergebnisse dieser Tests belegen, dass ein erfindungsgemäßer Extrakt der Wurzeln von Baptisia tinctoria den Effekt von UVB-Strahlung auf die Anzahl an Keratinocyten erheblich reduziert. Es zeigt sich eine deutliche Verringerung des durch UVB an menschlichen Keratinocyten induzierten PGE2 - Gehaltes und eine deutliche Verringerung des Gehalts an freigesetzter LDH. Die antiinflammatorische Wirkung ist vergleichbar mit der der 0,03 % igen Acetylsalicylsäure und beim 0,02 % igen Extrakt sogar besser als die von 0,001% Indomethacin. Der Baptisia tinctoria - Extrakt zeigt demnach die Fähigkeit, die durch UVB-Strahlung hervorgerufene Schädigung an Zellmembranen zu reduzieren und somit eine hemmende Wirkung gegen Entzündungen, die durch UVB Strahlung induziert werden.

### Gewerbliche Anwendung

Gegenstand der vorliegenden Erfindung sind ebenfalls kosmetische Zusammensetzungen für die lokale Anwendung auf der Haut, den Epithelanhanggebilden und/oder den Schleimhäuten, die als Wirkstoff alleine oder in Verbindung mit mindestens einem anderen Wirkstoff, mindestens eine Fraktion enthält, die aus Baptisia tinctoria-Wurzeln extrahiert wurde.

Diese kosmetische Zusammensetzung kann als einzigen Wirkstoff oder mit mindestens einem anderen Wirkstoff verbunden, mindestens einen Extrakt des vorab genannten Typs enthalten, der verwendet wird, um mindestens eine der besonderen biologischen Wirkungen zu erzeugen, die vorab beschrieben wurden oder sogar mehrere dieser Wirkungen als Kombination.

Die kosmetische Zusammensetzung der vorliegenden Erfindung kann zwischen 0,001 und 30 Gew. %, vorzugsweise zwischen 0,1 und 20 Gew. % und besonders bevorzugt zwischen 0,2 und 10 Gew. % eines Extraktes enthalten, der aus Baptisia tinctoria-Wurzeln extrahiert wurde (die Extrakte wurden durch eines der vorab erwähnten Verfahren gewonnen). Der Extrakt kann in geeignete kosmetische Vektoren, wie zum Beispiel Liposome, Makro-, Mikro- und Nanokapseln, Makro-, Mikro und Nanopartikel und andere analoge und bekannte Formen eingearbeitet werden.
Die vorab erwähnten Extrakte können für Anwendungen zur Pflege und Hygiene der Haut verwendet werden (Erzeugnisse für Gesicht und Körper, Tag- oder Nachtkosmetika, Sonnenschutzmittel, kräftigende, regenerierende Erzeugnisse, Anti-Faltenkosmetika, Schlankheitskuren-mittel, Mittel gegen Alterungsprozesse) in Form von Zubereitungen, wie zum Beispiel Lotionen oder Shampoos, Cremes, Schaummittel, Seifen, Stäbchen, Gele, Hydrogele, Sprühmittel, Emulsionen, Schutzmittel, reparierende, weichmachende, filmbildende und photoschützende Mittel ; Erzeugnisse für Dauerwellen und zur Haarfärbung.

Die vorab erwähnten Extrakte können zur Herstellung kosmetischer Mittel, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben, vornehmlich jedoch Erzeugnisse für Gesicht und Körper, Tag- oder Nachtkosmetika, Sonnenschutzmittel, kräftigende, regenerierende Erzeugnisse, Anti-Faltenkosmetika, Schlankheitskurenmittel und Mittel gegen Alterungsprozesse dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S, 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1),** insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
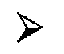 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
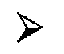 Alkyl- und/oder Alkenyloligoglykoside mit 8 bis 22 Kohlenstoffatomen im Alk(en)ylrest und deren ethoxylierte Analoga;
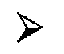 Anlagerungsprodukte von 1 bis 15 Mol Ethylenoxid an Ricinusöl und/oder gehärtetes Ricinusöl;
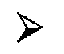 Anlagerungsprodukte von 15 bis 60 Mol Ethylenoxid an Ricinusöl und/oder gehärtetes Ricinusöl;
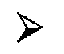 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
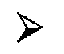 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
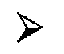 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
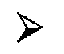 Mono-, Di- und Trialkylphosphate sowie Mono-, Di- und/oder Tri-PEG-alkylphosphate und deren Salze;
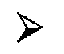 Wollwachsalkohole;
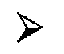 Polysiloxan-Polyalkyl-Polyether-Copolymere bzw. entsprechende Derivate;
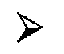 Block-Copolymere z.B. Polyethylenglycol-30 Dipolyhydroxystearate;
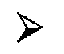 Polymeremulgatoren, z.B. Pemulen-Typen (TR-1,TR-2) von Goodrich;
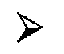 Polyalkylenglycole sowie
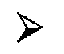 Glycerincarbonat.

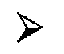 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht, C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

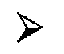 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

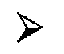 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

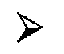 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

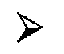 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

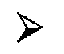 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

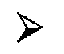 Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18-} Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### Weitere UV-Lichtschutzfilter und Antioxidantien

Neben den erfindungsgemäßen Extrakten der Baptisia tinctoria können weitere UV-Lichtschutzfaktoren eingesetzt werden. Diese sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
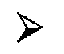 3-Benzylidencampher bzw. 3-Benzylidennorcampher und dessen Derivate, z.B. 3-(4-Methylbenzyliden)campher wie in der **EP 0693471 B1** beschrieben;
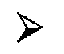 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
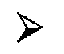 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
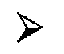 Ester der Salicylsäure, vorzugsweise Salicylsäure-2-ethylhexylester, Salicylsäure-4-iso-propylbenzylester, Salicylsäurehomomenthylester;
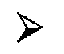 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
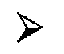 Ester der Benzalmalonsäure, vorzugsweise 4-Methoxybenzmalonsäuredi-2-ethylhexyl-ester;
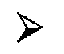 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
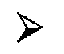 Propan-1,3-dione, wie z.B. 1-(4-tert.Butylphenyl)-3-(4'methoxyphenyl)propan-1,3-dion;
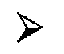 Ketotricyclo(5.2.1.0)decan-Derivate, wie in der **EP 0694521 B1** beschrieben.

Als wasserlösliche Substanzen kommen in Frage:
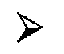 2-Phenylbenzimidazol-5-sulfonsäure und deren Alkali-, Erdalkali-, Ammonium-, Alkylammonium-, Alkanolammonium- und Glucammoniumsalze;
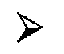 Sulfonsäurederivate von Benzophenonen, vorzugsweise 2-Hydroxy-4-methoxybenzo-phenon-5-sulfonsäure und ihre Salze;
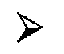 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543** (**1996**) sowie **Parf.Kosm. 3, 11** (**1999**) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, weitere Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

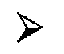 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

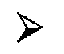 Enzyminhibitoren

Als Enzyminhibitoren sind beispielsweise Esteraseinhibitoren geeignet, Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

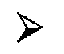 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

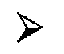 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
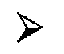 adstringierende Wirkstoffe,
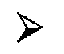 Ölkomponenten,
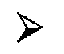 nichtionische Emulgatoren,
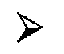 Coemulgatoren,
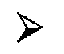 Konsistenzgeber,
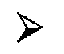 Hilfsstoffe wie z. B. Verdicker oder Komplexierungsmittel und/oder
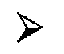 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z.B. mit Propylenglycol-1,2. Aluminiumhydroxyallantoinat, Aluminiumchloridtartrat, Aluminium-Zirkonium-Trichlorohydrat, Aluminium-Zirko-niumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
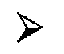 entzündungshemmende, hautschützende oder wohlriechende ätherische Öle,
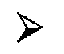 synthetische hautschützende Wirkstoffe und/oder
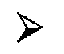 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
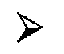 Glycerin;
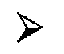 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
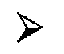 technische Oligoglyceringemische mit einem Eigenkondensationsgrad von 1,5 bis 10 wie etwa technische Diglyceringemische mit einem Diglyceringehalt von 40 bis 50 Gew.-%;
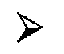 Methyolverbindungen, wie insbesondere Trimethylolethan, Trimethylolpropan, Trimethylolbutan, Pentaerythrit und Dipentaerythrit;
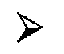 Niedrigalkylglucoside, insbesondere solche mit 1 bis 8 Kohlenstoffen im Alkylrest, wie beispielsweise Methyl- und Butylglucosid;
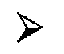 Zuckeralkohole mit 5 bis 12 Kohlenstoffatomen, wie beispielsweise Sorbit oder Mannit,
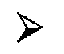 Zucker mit 5 bis 12 Kohlenstoffatomen, wie beispielsweise Glucose oder Saccharose;
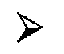 Aminozucker, wie beispielsweise Glucamin;
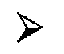 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.
Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000), Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Die Herstellung der Mittel kann durch übliche Kalt - oder Heißprozesse erfolgen; vorzugsweise arbeitet man nach der Phaseninversionstemperatur-Methode.

### Rezepturbeispiele

Als nicht einschränkende Beispiele werden nachstehend verschiedene Beispiele für die praktische Ausführung kosmetischer Zusammensetzungen nach der Erfindung beschrieben.

### BEISPIEL 1

Regenerationscreme - (dest. Wasser ad 100,0, Mengenangaben in Gew.%)

| Fettphase | |
|---|---|
| Ceteareth 25 | 2,00 |
| Ceteareth 6 und Stearylalkohol | 1,00 |
| Cetylalkohol | 4,00 |
| Glycolstearat | 4,00 |
| Vaseline | 5,00 |
| MCT- Triglyzeride, Miglyol | 5,00 |
| | |

| Wässrige Phase | |
|---|---|
| Glycerin | 10,00 |
| Baptisia tinctoria Extrakt nach Beispiel 1 | 2,00 |
| destilliertes Wasser | 8,50 |
| Elestab 4112 Konservierungsmittel (Laboratoires Sérobiol.) | 0,40 |
| Parfum | q.s. |

Die Fettphase wird bei 80° C aufgeschmolzen. Die wässrige Phase wird ebenfalls auf 80° C erhitzt und Elestab 4112 darin aufgelöst. Der Baptisia tinctoria - Extrakt wird separat vorbereitet, die fette Phase in die wässrige Phase unter Turbinenrühren gegeben, und danach bei ungefähr 50° C der Extrakt hinzugefügt. Danach wird mit dem Rühren bis zur Abkühlung fortgefahren.

### BEISPIEL 2

Creme für empfindliche Hauttypen und zur Behandlung von endzündlichen oder wunden Bereichen (dest. Wasser ad 100,0, Mengenangaben in Gew.%)

| Fettphase | |
|---|---|
| Glycolstearat | 14,00 |
| Octyl-Dodecanol | 6,00 |
| Dibutyl-Adipat | 6,00 |
| Ceteareth 12 | 1,50 |
| Ceteareth 20 | 1,50 |
| | |

| Wässrige Phase | |
|---|---|
| PVP (Polyvinylpyrrolidon) | 0,50 |
| Glycerin | 4,00 |
| Elestab 388 (Laboratoires Serobiologiques) | 2,00 |
| Baptisia tinctoria - Extrakt nach Beispiel 1 | 3,00 |
| destilliertes Wasser | 9,00 |
| Parfum | 0,20 |

Die Fettphase wird bei 80° C aufgeschmolzen. Die wässrige Phase wird ebenfalls auf 80° C erhitzt und Elestab 388 und PVP darin aufgelöst. Baptisia tinctoria - Extrakt wird separat vorbereitet, die fette Phase in die wässrige Phase unter Turbinenrühren gegeben, und danach bei ungefähr 50° der Extrakt hinzugefügt. Danach wird mit dem Rühren bis zur Abkühlung fortgefahren.

In den Tabellen 5a und 5b sind weitere Vorschläge für Rezepturen mit Baptisia tinctoria- Extrakt (Laboratoires Sérobiologiques) beschrieben.

**Tabelle 5a**

| **Kosmetische Zubereitungen (Mengenangaben in Gew.-%)** | | | |
|---|---|---|---|
| **Zusammensetzung (INCI)** | **1** | **2** | **3** |
| **Emulgade® SE** Glyceryl Sterate (and) Ceteareth 12/20 (and) Cetearyl Alcohol (and) Cetyl Palmitate | 5,0 | 5,0 | - |
| **Lameform® TGI** Polyglyceryl-3 Isostearate | - | - | 4,0 |
| **Monomuls® 90-O 18** _Glyceryl Oleate | - | - | 2,0 |
| **Cetiol® OE** Dicaprylyl Ether | - | - | 5,0 |
| **Cetiol® PGL** Hexyldecanol (and) Hexyldecyl Laurate | - | - | 10,0 |
| **Cetiol® SN** Cetearyl Isononanoate | 3,0 | 3,0 | - |
| **Cetiol® V** Decyl Oleate | 3,0 | 3,0 | - |
| **Myritol® 318** Coco Caprylate Caprate | - | - | 5,0 |
| **Bees Wax** | - | - | 7,0 |
| **Nutrilan® Keratin W** Hydrolyzed Keratin | 40,0 | 60,0 | - |
| **Gluadin® WK** Sodium Cocoyl Hydrolyzed Wheat Protein | - | - | 5,0 |
| **Magnesium Sulfate Hepta Hydrate** | - | - | 1,0 |
| **Glycerin** (86 Gew.-%ig) | 3,0 | 3,0 | 5,0 |
| **Baptisia tinctoria - Extrakt nach Beispiel 1** | 2,0 | 5,0 | 3,0 |
| **Aqua conservata** | ad 100,0 | ad 100,0 | ad 100,0 |
| (1) Softcreme, (2) Feuchtigkeitsemulsion, (3) Nachtcreme | | | |

**Tabelle 5b**

| **Kosmetische Zubereitungen (Mengenangaben in Gew.-%)** | | | | |
|---|---|---|---|---|
| **Zusammensetzung (INCI)** | **4** | **5** | **6** | **7** |
| **Dehymuls® PGPH** Polyglyceryl-2 Dipolyhydroxystearate | 2,0 | 3,0 | - | - |
| **Lameform® TGI** Polyglyceryl-3 Diisostearate | 4,0 | 1,0 | - | - |
| **Eumulgin VL 75** Polyglyceryl-2 Dipolyhydroxystearate (and) Lauryl Glucoside (and) Glycerin | - | - | 3,5 | - |
| **Bees Wax** | 3,0 | 2,0 | - | - |
| **Cutina® GMS** Glyceryl Stearate | - | - | 2,0 | 4,0 |
| **Lanette® O** Cetearyl Alcohol | - | - | 4,0 | 1,0 |
| **Antaron® V 216** PVP / Hexadecene Copolymer | - | - | 3,0 | 2,0 |
| **Plantaren® 818** Cocoglycerides | 5,0 | - | 6,0 | 5,0 |
| **Finsolv® TN** C12/15 Alkyl Benzoate | - | 6,0 | - | 2,0 |
| **Dioctyl Carbonate** | 5,0 | 4,0 | 5,0 | 6,0 |
| **Cetiol® J 600** Oleyl Erucate | 2,0 | - | 3,0 | 4,0 |
| **Cetiol® OE** Dicaprylyl Ether | 3,0 | - | 1,0 | - |
| **Mineral Oil** | - | 4,0 | 2,0 | - |
| **Cetiol® PGL** Hexadecanol (and) Hexyldecyl Laurate | - | 7,0 | - | - |
| **Panthenol / Bisabolol** | 1,2 | 1,2 | 1,2 | 1,2 |
| **Copherol® F 1300** Tocopherol / Tocopheyl Acetate | 0,5 | 1,0 | 1,0 | 2,0 |
| **Neo Heliopan® Hydro** Sodium Phenylbenzimidazole Sulfonate | 3,0 | - | - | - |
| **Neo Heliopan® 303** Octocrylene | - | 5,0 | 4,0 | 10,0 |
| **Neo Heliopan®** BB Benzophenone-3 | 1,5 | - | - | - |
| **Neo Heliopan® E 1000** Isoamyl p-Methoxycinnamate | 5,0 | - | 2,0 | - |
| **Neo Heliopan® AV** Octyl Methoxycinnamate | 4,0 | - | 3,0 | 2,0 |
| **Uvinul® T 150** Octyl triazone | 2,0 | 4,0 | 1,0 | 3,0 |
| Zinc Oxide | - | 6,0 | - | 5,0 |
| **Titanium Dioxide** | - | 2,0 | - | - |
| **Glycerin (86 Gew.-%ig)** | 5,0 | 5,0 | 5,0 | 5,0 |
| **BAPTISIA TINCTORIA- Extrakt nach Beispiel 1** | 3,0 | 2,0 | 5,0 | 4,0 |
| **Aqua conservata** | ad 100,0 | ad 100,0 | ad 100,0 | ad 100,0 |
| (4) W/O-Sonnenschutzcreme, (5) W/O-Sonnenschutzlotion, (6) O/W-Sonnenschutzcreme (7) O/W-Sonnenschutzlotion | | | | |

## Patentansprüche

1. Verwendung eines aus den Wurzeln von Baptisia tinctoria extrahierten Extraktes zur Herstellung kosmetischer Zubereitungen für die lokale Anwendung auf der Haut, den Epithelanhanggebilden und/oder den Schleimhäuten.

2. Verwendung nach Anspruch 1 zur Herstellung von Sonnenschutzmitteln.

3. Verwendung nach Anspruch 1 zur Herstellung von Mitteln mit Anti-Ageing Aktivitäten.

4. Verwendung nach Anspruch 1 zur Herstellung von Mitteln mit östrogenähnliche Aktivitäten.

5. Verwendung nach Anspruch 1 zur Herstellung von Mitteln mit anti-inflammatorischen Aktivitäten.

6. Verwendung nach Anspruch 1 zur Herstellung von Anti-Akne-Mitteln.

7. Verwendung nach Anspruch 1 zur Herstellung von Mitteln mit Schutzfunktion gegen oxidativen Stress und Umweltgifte.

8. Verwendung nach Anspruch 1 zur Herstellung von Mitteln zur Stimulation der Synthese von Makromolekülen ausgewählt aus der Gruppe, die gebildet wird von Glucosaminoglucanen, Hyaluronsäure, Chondroitinsulfat, Dermatansulfat, Keratansulfat, Proteoglycanen, Kollagen und Elastin.

9. Verwendung nach Anspruch 1 zur Herstellung von Mitteln zur Behandlung empfindlicher Hauttypen.

10. Verwendung nach Anspruch 1, **dadurch gekennzeichnet, dass** ein Extrakt aus den Baptisia tinctoria-Wurzeln als Wirkstoff eingesetzt wird, der gegen Schädigungen an Keratinocyten und Fibroblasten durch UV-Bestrahlung, insbesondere UV-A- und/oder UV-B-Bestrahlung, wirksam ist.

11. Verwendung nach Anspruch 1, **dadurch gekennzeichnet, dass** ein Extrakt aus den Baptisia tinctoria-Wurzeln als Wirkstoff eingesetzt wird, der zellschützende Eigenschaften aufweist.

12. Verwendung nach Anspruch 1 zur Herstellung von Mitteln mit fettabbauender Aktivität und Anticellulitiswirkung.

13. Verfahren zur Herstellung eines Extrakts aus der Pflanze Baptisia tinctoria **dadurch gekennzeichnet, dass** mit einem Extraktionsmittel ausgewählt wird aus der Gruppe, die gebildet wird von Wasser, niedrigmolekularen Alkoholen, Estern, Ketonen, Kohlenwasserstoffen und halogenierten Kohlenwasserstoffen, extrahiert wird und gegebenenfalls nachfolgend getrocknet wird.

14. Verfahren zur Herstellung nach Anspruch 12, **dadurch gekennzeichnet, dass** der Extrakt durch Wasser oder Wasser/Alkoholgemische extrahiert wird.

15. Verfahren zur Herstellung eines Extraktes aus der Pflanze Baptisia tinctoria, **dadurch gekennzeichnet, dass** primäre und sekundäre Metaboliten durch Kultivierung der Pflanze unter Hydroponie-Bedingungen gewonnen werden.

16. Kosmetische Zubereitungen für die lokale Applikation, enthaltend einen Extrakt aus den Wurzeln der Pflanze Baptisia tinctoria.

17. Kosmetische Zubereitungen enthaltend mindestens eine Substanz, ausgewählt aus der Gruppe die gebildet wird aus Isoflavonen, Flavonen, Proteinen, Glycoproteinen und Kohlenhydraten, die extrahiert sind aus den Wurzeln der Pflanze Baptisia tinctoria.

18. Kosmetische Zubereitungen nach mindestens einem der Ansprüche 16 und/oder 17, **dadurch gekennzeichnet, dass** sie 0,001 bis 30 Gew. % eines Extraktes, der aus Baptisia tinctoria extrahiert wurde, enthalten.
